# EUROPEAN PATENT SPECIFICATION

(11) **EP 1 854 408 B1**
(45) Date of publication and mention of the grant of the patent: **27.10.2010**
(21) Application number: 07014058.7
(22) Date of filing: 16.08.2002
(51) Int. Cl.: A61B 5/15, A61B 5/00

(54) **In-situ adapter for an analyte testing device**
In-situ-Adapter für eine Testvorrichtung
Adaptateur in-situ pour dispositif de test

(30) Priority: 16.08.2001 US 313059 P
(43) Date of publication of application: 14.11.2007
(62) Divisional of application: 02758541.3
(73) Proprietor: Lifescan Scotland Ltd, Inverness IV2 3ED (GB)
(72) Inventor: Moerman, Piet, 9831 St. Martens-Latem (BE); Griffith, Alun, Inverness IV2 3XQ (GB)
(74) Representative: Tunstall, Christopher Stephen

(56) References cited:
- WO-A-01/28423
- WO-A-97/42882
- WO-A-99/27854
- US-A- 4 895 147
- US-A- 5 871 494

## Description

### FIELD OF THE INVENTION

The present invention relates to an adapter for a testing device for sampling and analyzing analytes in bodily fluids, in particular a glucose meter for sampling and analyzing glucose in blood or interstitial fluid.

### BACKGROUND OF THE INVENTION

Glucose monitoring is a fact of everyday life for diabetic individuals. The accuracy of such monitoring can significantly affect the health and ultimately the quality of life of a person with diabetes. Generally, a diabetic patient measures blood glucose levels several times a day to monitor and control blood sugar levels. Failure to test blood glucose levels accurately and on a regular basis can result in serious diabetes-related complications, including cardiovascular disease, kidney disease, nerve damage and blindness. A number of glucose meters are currently available which permit an individual to test the glucose level in a small sample of blood.

Many of the glucose meter designs currently available make use of a disposable test strip, which, in combination with the meter, electrochemically measures the amount of glucose in the blood sample. To use these meters, the user first pricks a finger or other body part using a separate lancing device to produce a small sample of blood or interstitial fluid. The sample is then transferred to a disposable test strip having an electrode system. The test strip is then inserted into the glucose meter to measure the glucose level of the sample. The test strip may be inserted into the meter prior to obtaining a blood sample. The inconvenience of taking several measurements a day as well as the pain inflicted by currently available lancets or finger-sticking devices often discourage disciplined and frequent testing.

WO 01/28423 discusses a larcing device and method for self-collecting a sample of body fluid by penetrating a body tissue at a sampling site.

While the fingertip is generally used for sampling blood, due to the rich capillary bed of the skin of the fingertip, the fingertip is also particularly sensitive to pain, due to a rich supply of pain receptors in the fingertip as well. When the incision from a lancet is too deep, too close to a recent incision or not deep enough and requires an additional incision, the pain from a puncture is increased significantly. Pain may also be increased if the cutting blade penetrates slowly or is withdraw slowly. Furthermore, the user may he forced to make a larger incision than is necessary to form a sufficient amount of blood, due to losses incurred during the transfer of the sample between the puncture site and the test strip.

The process of monitoring blood glucose levels requires several steps and several different accessories, including a lancing device, a lancet, a supply of test strips and a glucose meter. Each accessory has a different function. The user must have a flat surface available to unpack and lay down the accessories within easy reach. First, the user charges the lancing device with a fresh lancet. Then, the user opens a vial of strips, removes a strip and inserts a strip into the meter. The user then re-closes the vial and checks for the correct calibration code on the meter. Then the user picks up the lancing device and lances the skin of the finger or other body part. The user then lays down the lancing device and squeezes or massages the finger to yield an adequate blood sample. The user then transfers the sample to a test strip for analysis. Generally, a user is required to transfer a specific volume of sample to a specific location on the small test strip, a difficult task for many users. After transfer of the sample, the user waits for the meter to analyze the sample, then removes the strip from the test meter and discards the strip. Finally, the user re-packs all of the accessories. As set forth above, the standard regime for monitoring glucose requires the use of multiple, separate components.

The pain, inconvenience, cost, slowness, complexity and discreteness of running a blood test are barriers to the frequent monitoring of glucose glucose levels. Patients often do not comply with doctor recommendations to frequently test glucose levels due to the numerous obstacles involved.

### SUMMARY OF THE INVENTION

In view of the foregoing and in accordance with one aspect af the present invention, there is provided a lancet device comprising:
a lancet for puncturing the skin of a user;
a lancet drive train for holding and driving the lancet into the skin of a user, the lancet drive train having a cocked position and a stopping position;
a cocking button for cocking the lancet drive train; and
a depth adjustment mechanism for adjusting the penetration depth of the lancet, the depth adjustment mechanism comprising:
   a substrate adapted to be movably mounted in a slot of a housing of the lancet device and having an interior surface, wherein the position; of the substrate in the slot defines the cocked position and the stopping position;
   a first projection on the interior surface of the substrate, the first projection adapted to interface with the lancet drive train to define the cocked position; and
   a second projection on the interior surface of the substrate substantially parallel to the first projection, the second projection adapted to interface with a cocking button to define the stopping position.

Preferably, the first projection is adapted to interface with a catch on the lancet drive train.

Preferably, the cocking button includes a stop and the second projection is adapted to interface with the stop.

Preferably, the depth adjustment mechanism further comprises a rim disposed on the interior side, wherein the rim is adapted to interface with a groove on the housing to allow movement of the substrate.

Preferably, the depth adjustment mechanism further comprises a knob disposed on an external surface of the substrate to allow a user of the lancet device to move the substrate in the slot.

Preferably, the cocking button includes a cocking rib adapted to interface with the second projection and an interior rib defining a stop for lancet drive train.

Preferably, the lancet device further comprises a catch on the lancet drive train adapted to interface with the first projection to define a cocked position of the lancet drive train.

In accordance with a second aspect of the present invention, there is provided an integrated testing device for testing analytes in bodily fluids comprising the lancet device according to the first aspect of the invention.

In accordance with a third aspect of the present invention, there is provided an integrated testing device for testing analytes in bodily fluids comprising:
a housing;
a test port for holding a test strip;
a testing device for testing analytes in bodily fluids applied to the test strip;
a housing connector for connecting the housing to the testing device; and the lancet device according to the first aspect of the invention.

Preferably, the housing connector comprises:
a removeable housing surface; and
u projection on the housing surface,
wherein the projection is configured for connection with a housing feature on the testing device, such that the connector connects the adapter to the testing device.

### BRIEF DESCRIPTION OF THE DRAWINGS

These and other features and advantages of the present invention will be more fully understood by reference to the following detailed description in conjunction with the attached drawings in which like reference numerals refer to like elements through the different views.

A specific embodiment is now described by way of example only and with reference to the accompanying drawings, in which:
Figure 1 is a general view of the adapter of the illustrative embodiment of the invention attached to a standard glucose meter;
Figure 2 is a side view of the adapter and glucose meter assembly of Figure 1;
Figure 3 is a general view of the adapter and glucose meter assembly of Figure 1;
Figure 4 illustrates the adapter wherein a half of the housing and the cap are removed to expose the interior of the adapter;
Figure 5 is an exploded view of the lancing mechanism in the device;
Figure 6 illustrates the lancet barrel of the adapter;
Figures 7a and 7b illustrative the details of the depth adjustment button;
Figure 8 illustrates the lancing mechanism in a cocked position the cocking button removed for clarity;
Figures 9a and 9b illustrate the details of the cocking button of the adapter;
Figure 10 illustrates the assembled lancing mechanism during a cocking process;
Figure 11 is a general partial view of the adapter, illustrating the connector for connecting the adapter to a glucose meter; and
Figures 12a, 12b, 12c and 12d illustrate the strip contact extension feature for extending electrical contact from a test strip in the adapter to the glucose meter electronics.

### DETAILED DESCRIPTION OF THE ILLUSTRATIVE EMBODIMENT

The present invention is described below relative to an illustrative embodiment. Those skilled in the art will appreciate that the present invention may be implemented in a number of different applications and embodiments and is not specifically limited in its application to the particular embodiment depicted herein. The present invention is discussed below in connection with sampling blood for analysis by a glucose meter, although those of ordinary skill will recognize that other types of testing devices could be used for sampling and testing other fluids.

There is now described an illustrative embodiment of an adapter. The adapter is for use with a glucose meter to provide an integrated device for obtaining and analyzing a sample of bodily fluid, such as blood. The adapter facilitates the monitoring of blood glucose levels by providing a means for integrating into a single device the steps involved in sampling and analyzing blood. The integration results in a simplified process employing a single device.

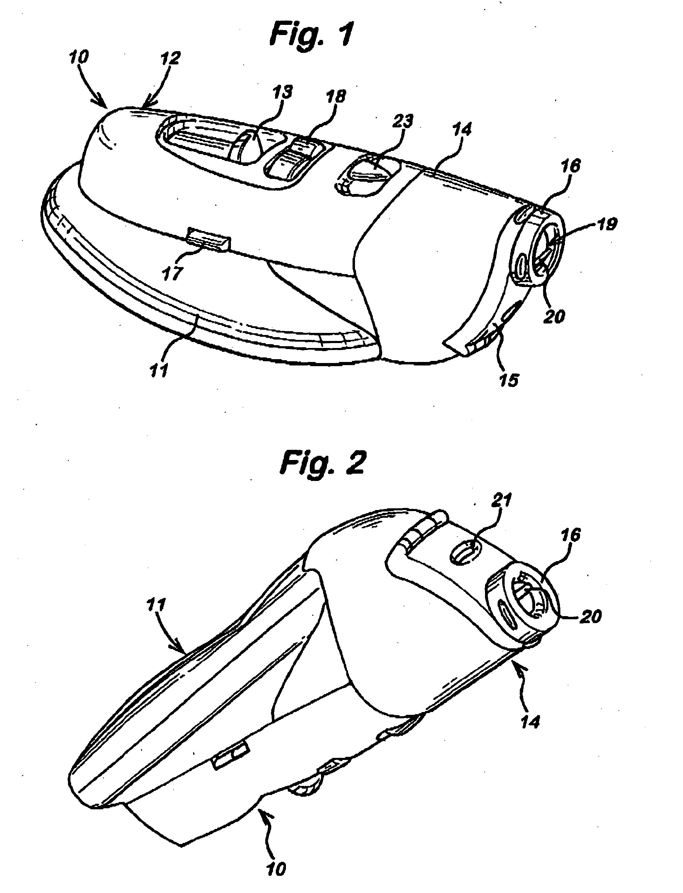

Figure 1 illustrates a clip-on adapter device 10 of the illustrative embodiment of the present invention. The adapter device 10 is attached to a standard glucose meter 11 to convert the glucose meter into an integrated device for sampling and testing a blood sample. The adapter device 10 includes a housing 12, a lancing mechanism (not shown), including a lancet for puncturing the skin of a user to express a drop of blood, a cocking button 13 for cocking the lancing mechanism and a depth adjustment button 18 for adjusting the penetration depth of the lancet. As shown, the cocking button 13 is slidably mounted in the housing and protrudes through longitudinal slot in the housing. According to the illustrative embodiment, a user retracts the cocking button towards the distal end of the housing to cock the lancing mechanism. The depth adjustment button 18 is rotatably mounted in the housing and protrudes through a second longitudinal slot in the housing. The user rotates the depth adjustment button 18 about its axis to vary the penetration depth of the lancet. After the user cocks the lancing mechanism and selects a penetration depth, the user depresses a triggering button 23 to release the lancet from the cocked position. A housing connector 17 connects the adapter 10 to the glucose meter 11 to form an integrated sampling and testing device. According to the illustrative embodiment, the housing connector 17 replaces the original battery door of the glucose meter and has the dual function of enclosing a battery compartment in the glucose meter and providing a connection to the adapter 10.

A removable lancet cap 14 is attached to the proximal end of the housing and includes an aperture 19 to allow passage of the lancet through the cap and into the skin of the user. According to the illustrative embodiment, the cap is substantially clear or includes a substantially clear portion to allow a user to view a sample being expressed from the puncture site when the lancet punctures the skin of the user. The cap 14 includes a pressure ring 16 disposed about the aperture 19. The pressure ring 16 has a multi-contoured surface in order to promote, enhance or facilitate the expression of blood by pressing the device onto the skin. A hinged door 15 provides access to the interior of the cap 14. According to the illustrative embodiment, a storage compartment (not shown) is provided within the interior of the cap 14, which allows a user to store spare lancets, pressure rings, test strips and the like.

The cap 14 for the adapter includes a pressure ring 16 attached to the distal end of the cap body. As discussed, the pressure ring 16 is mounted around and encloses the aperture 19 through which the lancet passes. The pressure ring 16 has a multi-contoured surface oriented generally about an axis distinct from the axis of motion of the lancet. The multi-contoured surface is designed to exert pressure on the dermal tissue to facilitate expression of a fluid sample after lancing the dermal tissue. According to an alternate embodiment, the pressure ring 16 comprises a pair of pressure wings sized and dimensioned to accommodate the sharp curve of the fingertip therebetween. The pressure wings thus form a recess for accommodating the finger of the user. This applies the correct amount of pressure to allow for the expression of blood.

A test strip 20 is positioned in an aperture 19 or a test port (not shown) and extends through the aperture 19. When the lancet punctures the skin and a drop of blood forms on the skin surface, the position of the test port locates the test strip in close proximity to receive the blood sample. The adapter further includes an extension (not shown) to the test port of the glucose meter 11, which extends the electrical contacts from the test port of the glucose meter to the contacts on the test strip 20. In this manner, in situ testing of the blood sample is enabled. The individual features and components of the adapter device 10 will be described in more detail below.

The adapter device 10 of the illustrative embodiment is configured to clip onto a standard glucose meter, such as a One Touch Ultra meter from Lifescan, the QID meter from Abbott, the Glucometer Elite meter from Bayer, the Advantage meter from Roche, and other commercially available glucose meters. The housing connector 17, which enables the attachable clip-on feature, may be customized for any suitable particular glucose meter employed by the user.

Figure 2 illustrates a side view of the adapter device 10 connected to the glucose meter 11 and including an alternative access port 21. As illustrated, the alternative access port 21 is provided in the hinged door 15. The alternative access port 21 accepts an alternative test strip such that contacts on the alternative test strip connect to the electrical contacts from the test strip port. The alternative access port 21 does not have a surrounding pressure ring 16 or lancet adjacent the alternative access port 21. This way, a test strip can be placed in the alternative access port 21, the skin of a user can be pricked with an independent lancing device to obtain a blood sample, and the blood sample can be applied to the test strip for analysis by the glucose meter 11.

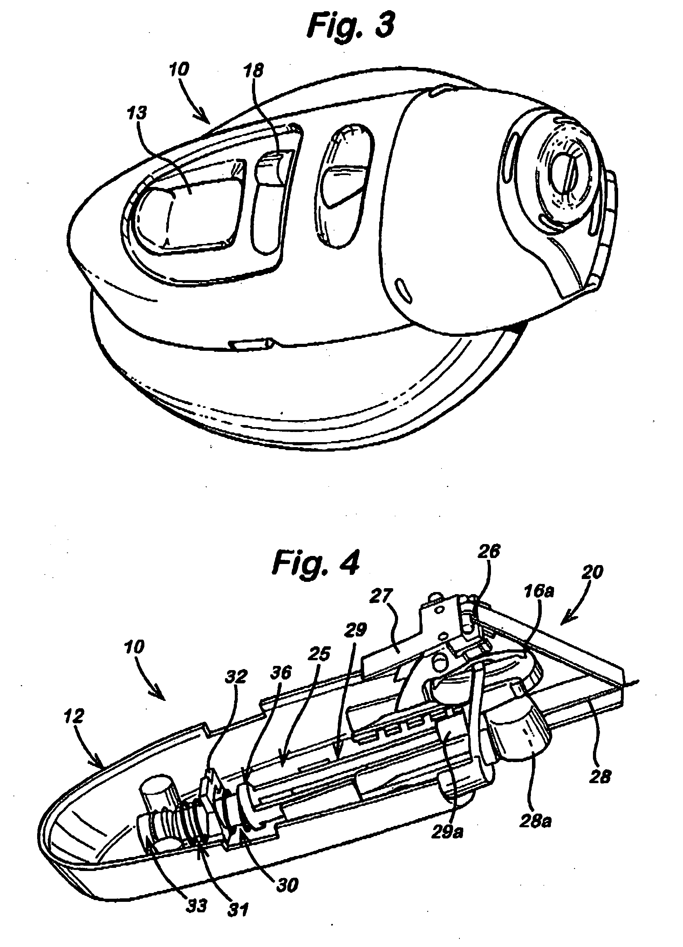

Figure 3 is a general view of the integrated device comprising the adapter 10 connected to the glucose meter 11. In Figure 3, the cocking button 13 is shown in a retracted position, corresponding to the end of a cocking process. The depth adjustment button 18 shown in Figure 3 is rotated to select a minimum puncture depth. As discussed, the puncture depth is determined by the position of the depth adjustment button, and the puncture depth is adjusted by rotating the button between a variety of positions. According to the illustrative embodiment, the depth adjustment button is rotated to the left side of the longitudinal slot to select a minimum depth and the right side of the longitudinal slot to select a maximum penetration depth. One skilled in the art will recognize that the depth adjustment feature is not limited to the illustrative embodiment. For example, the depth adjustment button 18 may be configured such that rotation to the right will decrease the penetration depth. Alternatively, the depth adjustment button may be configured such that a linear movement in a predetermined direction alters the penetration depth of the lancet.

Figure 4 illustrates the adapter 10 wherein a half of the housing 12 and the cap 14 are removed to expose the interior of the adapter 10. The interior of the adapter houses the lancing mechanism 25, a test strip 20, a set of electrical contact bars 26 and a printed circuit board 27 for connecting the test strip to the glucose meter. The lancing mechanism is slidably mounted within the housing of the adapter 10 and includes a disposable lancet 28 and a lancet drive train for driving the lancet into and out of the skin of a user. The lancet drive train comprises a lancet barrel 29 having a barrel collar 36 and a barrel base 33, a first spring 30, a second spring 31 and a spring retainer 32. The lancet 28 is releasably mounted in the proximal end 29a of the lancet barrel 29. The lancet barrel is connected to the cocking button (not shown in Figure 4), such that retraction of the cocking button towards the distal end of the adapter will retract the lancet barrel to a cocked position. In addition, Figure 4 illustrates the implementation of storage compartments within the housing of the adapter 10 for storing a spare lancet 28a and a spare or alternate pressure ring 16a.

The first spring 30 comprises a relatively strong spring for driving the lancet. The first spring 30 is housed between the barrel collar 36 and the spring retainer 32 and is compressed when the lancing mechanism is retracted to the cocked position. According to the illustrative embodiment, the amount of compression in the first spring 30 when the lancet is fully cocked depends on the position of the depth selection button, which will be described in detail below. The first spring 30 has a restoring force that is proportional to the amount of compression. When the lancet barrel is released from the cocked position by depressing the trigger button, the restoring force propels the lancet along a predetermined path through the aperture 19 of the adapter. The second spring 31 comprises a relatively weak spring and is housed between an interior rib of the cocking button (not shown in Figure 4) and the barrel base 33. The weak spring 31 is also compressed as the cocking button is retracted to cock the lancet. When the user releases the cocking button, the weak spring biases the cocking button back to a rest position, while the lancet barrel remains in a locked cocked position.

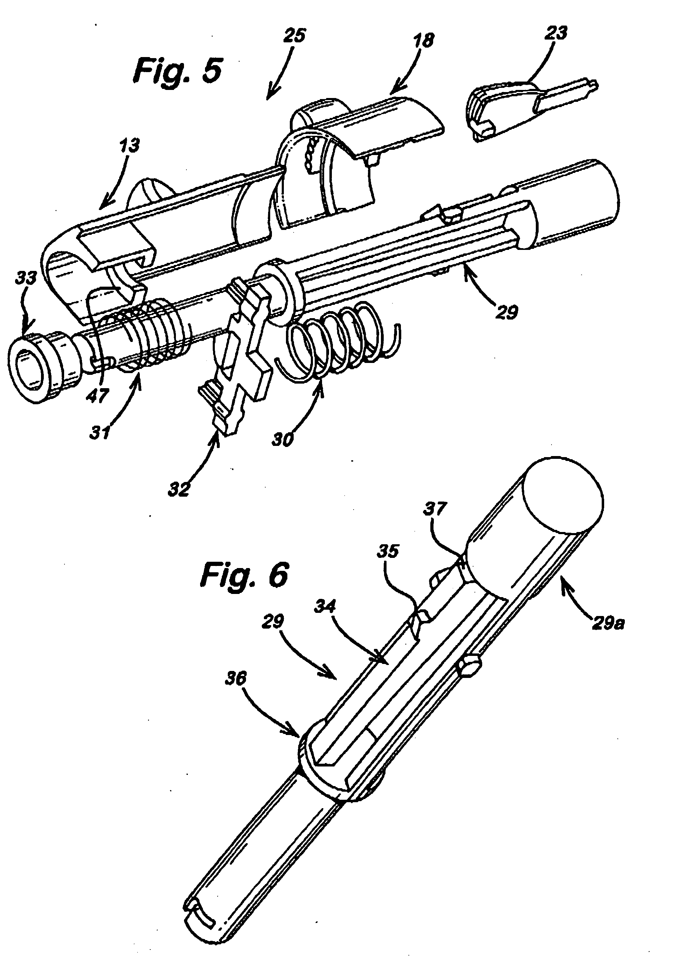

Figure 5 is an exploded view of the lancing mechanism 25, illustrating the different components and the relative positions of the respective components involved in the lancing and depth adjustment functions. The trigger button 23, depth adjustment button 18 and the cocking button 13 are arranged relative to each other and the lancet barrel 29 and cooperate to arm the lancing mechanism, fire the lancing mechanism and control the penetration depth of the lancet. As discussed, the cocking button 13 operates to retract the lancet barrel 29 from a rest position to a cocked position. The barrel is temporarily locked in the cocked position by a rib 41 located on the interior side of the depth adjustment button 18. The position of the depth adjustment button 18 determines the starting position (i.e. the cocked position) of the lancet 28 before the lancet is released and penetrates the skin. The trigger button 23 releases the barrel 29 from the cocked position when a user depresses the trigger button. Upon release, the lancet travels a predetermined distance until the barrel base 33 abuts an internal rib 47 of the cocking button, which stops the lancet travel. The first spring 30 then retracts the lancet from the skin. The working and functionality of the different components, particularly the lancet barrel 29, the cocking button 13, the depth adjustment button 18 and the trigger button 23 will be described in detail below.

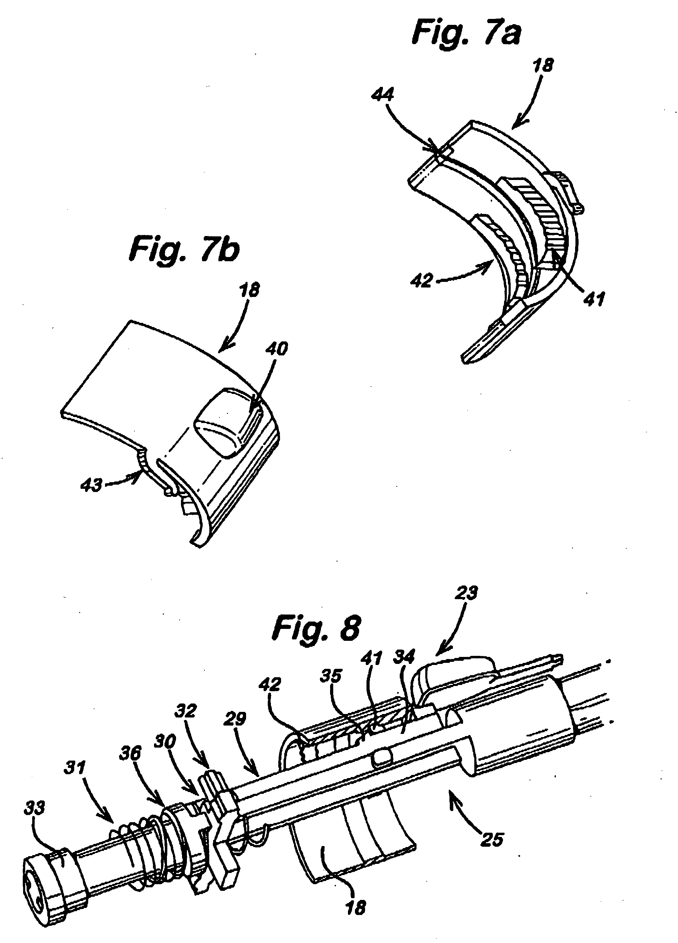

Figure 6 illustrates the lancet barrel 29 of the adapter 10 according to the illustrative embodiment. The proximal end 29a of the lancet barrel is configured to hold a lancet 28. The barrel 29 includes a flexible arm 34 and a catch 35 on the flexible arm for temporarily locking the barrel 29 in a cocked position. According to the illustrative embodiment, the catch 35 interfaces with a rib 41 on the depth adjustment button, shown in Figure 7 and described in detail below, to lock the barrel in the cocked position. According to the illustrative embodiment, the position of the cocked lancing mechanism is determined by the angular position of the depth adjustment button 18. The trigger button 23 cooperates with an end portion 37 of the flexible arm 34 to release the barrel 29 from the cocked position. When a user depressed the trigger button 23, the trigger presses the end portion 37, which forces the flexible arm 34 to flex, thereby releasing the catch 35 from the rib of the depth adjustment button 18.

Figures 7a and 7b illustrate the depth adjustment button 18 according to the illustrative embodiment. The depth adjustment button 18 allows a user to select a preferred penetration depth and customize the adapter to different people and different body parts. The depth adjustment button 18 comprises an arc-shaped substrate that is rotatably mounted in the housing 12 of the adapter and extends through a longitudinal slot in the housing 12, as illustrated in Figures 1-3. A knob 40 disposed on the exterior side of the depth adjustment button 18 may be grasped by a user to rotate the depth adjustment button about the longitudinal axis of the adapter 10 to select the desired penetration depth. A rim 44 on the depth adjustment button 18 cooperates with a groove in the housing to facilitate rotational movement of the depth adjustment button. The angular position of the depth adjustment button 18 determines the depth of the lancing mechanism. To increase the penetration depth, a user rotates the depth adjustment button in a first direction. To decrease the penetration depth, the user rotates the depth adjustment button in an opposite direction.

To provide the variable depth feature, a first rib 41 and a second rib 42 are disposed on an interior side of the depth adjustment button 18. According to the illustrative embodiment, the ribs 41 and 42 are slanted to allow selection of variable penetration depths, to provide smooth transition between different positions, and to accommodate the limited space underneath the surface of the depth adjustment button 18. The first rib 41 cooperates with the catch 35 on the flexible arm of the lancet barrel to define a cocked position of the lancet, as described above. As shown, the longitudinal position of any segment of the first rib 41 is controlled by the rotation of the button around its axis. The second rib 42 is parallel to the first rib and interfaces with a cocking rib 46 on the cocking button 13, to be described in detail below, to define the final penetration depth of the lancet. The cocking rib 46 on the cocking button 13 abuts the second rib 42 to define the rest position of the cocking button 13. The cocking button in turn defines a stop for the lancet mechanism, thereby controlling the penetration depth of the lancet 28. (The position of the depth adjustment button defines the position of the cocking button, which defines the stop position of the lancet). The rotation of the depth adjustment button 18 brings different portions of the ribs 41, 42 into the path of the catch of the lancet barrel 29 and the cocking rib of the cocking button 13, respectively. When rotated to the left, the rib interfaces (i.e. the starting and stopping positions of the lancet) are moved back towards the distal end of the adapter, decreasing the amount that the lancet protrudes from the aperture. When rotated to the right, the rib interfaces move forwards, towards the proximal end of the adapter, thereby increasing the amount that the lancet protrudes from the aperture. A depth selection arm 43 allows a user to select a discrete depth position when rotating the depth adjustment button 18 about its axis. The arm 43 "clicks" into each discrete depth position to indicate to the user that a particular depth has been selected. The depth adjustment feature provides a simplified and accurate means for ensuring that a desired penetration depth is achieved. One skilled in the art will recognize that the depth adjustment button is not limited to the illustrative configuration and that any suitable shape or arrangement may be utilized to select a penetration depth for a lancet and that the present invention is not limited to the illustrative embodiment.

Figure 8 illustrates the lancing mechanism in a cocked position. As shown, the depth adjustment button 18 holds the lancing mechanism in the cocked position. As the lancing mechanism, including the barrel 29, is retracted using the cocking button, the catch 35 of the barrel 29 is caught behind the first rib 41 of the depth adjustment button to temporarily lock the barrel in the cocked position. As shown, the position of the depth adjustment button 18 defines the starting position of the lancet, and rotation of the depth adjustment button varies the starting position of the lancet. In Figure 8, the depth adjustment button 18 is rotated to the left to move the starting position back towards the distal end of the adapter and thereby produce a shorter penetration depth. As discussed, the trigger 23 is depressed to flex the flexible arm 34 and release the lancet barrel catch from behind the first rib 41.

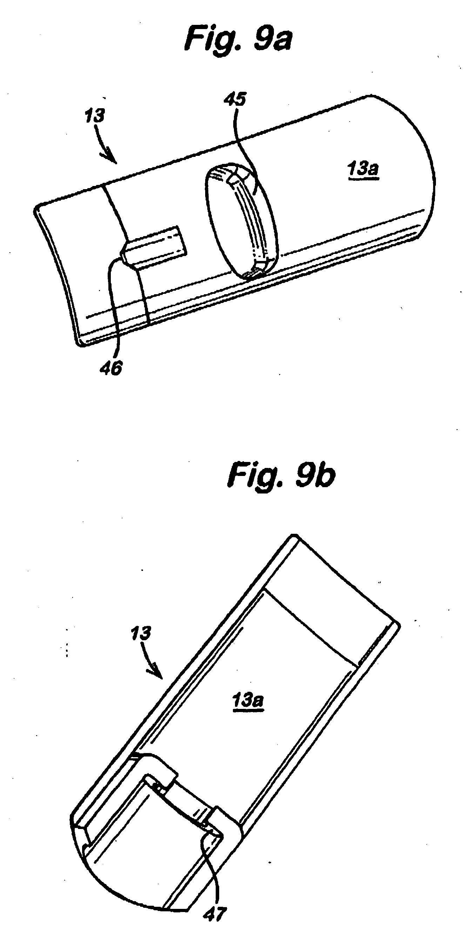

Figures 9a and 9b illustrate the cocking button 13 of the illustrative embodiment. As discussed, the cocking button 13 is slidably mounted in the housing 12 of the adapter 10 of the illustrative embodiment and extends through a longitudinal slot in the housing. The cocking button 13 of the illustrative embodiment comprises a substrate 13a, a cocking knob 45 and a cocking rib 46 disposed on the exterior surface of the substrate 13a and an interior rib 47 disposed on the interior surface of the substrate 13a. The cocking button 13 is connected to the lancing mechanism 25 and adapted to slide in the longitudinal slot.

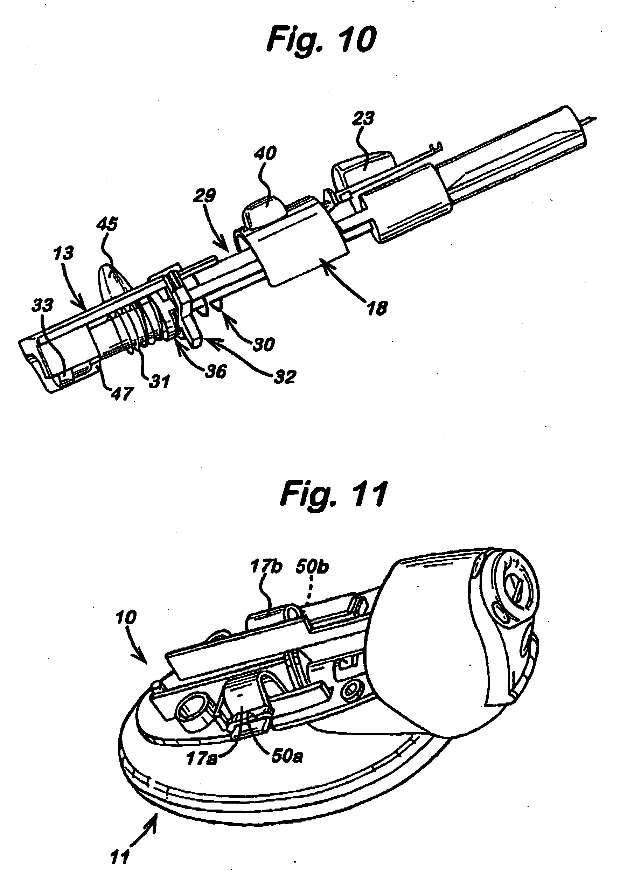

Figure 10 illustrates the assembled lancing mechanism, including the cocking button, the trigger button and the depth adjustment button during the cocking process. To cock the lancing mechanism, a user grasps the cocking knob 45 and slides the cocking button 13 towards the distal end of the adapter 10. The interior rib 47 on the cocking button 13 abuts the barrel base 33 and forces the barrel base 33 to retract with the cocking button. During the cocking process, the weak spring 31 becomes compressed between the interior rib 47 and the barrel base 33. The strong spring 30 becomes compressed between the barrel collar 36 and the spring retainer 32. The first rib of the depth adjustment button 18 captures the catch 35 of the lancet barrel 29 to hold the lancing mechanism in the cocked position, such that the strong spring remains compressed 30. The weak spring 31 of the lancing mechanism biases the cocking button back towards the proximal end of the adapter, until the cocking rib 46 on the exterior surface of the cocking button 13 abuts the second rib on the interior of the depth adjustment button 18. The interface between the cocking rib 46 and the cocking button 13 defines the rest position of the cocking button 13. The interior rib 47 of the cocking button includes an opening sized and dimensioned to allow the lancet barrel 29 to slide through while retaining the barrel base 33. The interior rib 47 serves as a stop for the lancet. When the drive train drives the lancet 28 through the aperture 19 in the adapter cap 14, the interior rib 47 stops lancet at a predetermined travel depth. As discussed, the position of the interior rib 47, and thus the penetration depth of the lancet, is defined by the position where the cocking rib 46 strikes the second slanted rib 42 of the depth adjustment button 18, which is in turn defined by the axial position of the depth adjustment button 18.

The lancing mechanism of the illustrative embodiment, including the system and method for adjusting the penetration depth, may be used alone, and is not limited to use in an adapter for a glucose meter, as illustrated. According to an alternate embodiment of the invention, the illustrated lancing mechanism may be utilized in a stand-alone lancet device that is not adapted to attach to a glucose meter, which incorporates the depth adjustment capabilities described herein.

Figure 11 is a general partial view of the adapter 10 of the present invention connected to a glucose meter 11. Figure 11 illustrates the attachment of the adapter 10 to the glucose meter using a housing connector 17 specially designed to integrate the adapter device with the meter. According to the illustrative embodiment, the housing connector 17 is configured to replace the original battery door of the meter. The housing connector 17 replicates the battery door and covers the batteries (not shown) in the glucose meter in the same way. The housing connector 17 further includes protrusions 17a and 17b, which are configured to secure the adapter 10 to the glucose meter 11. As shown, the adapter 10 includes slots 50a, 50b for receiving the protrusions 17a, 17b. One skilled in the art will recognize that any suitable means for releasably attaching the adapter device 10 to a glucose meter may be utilized.

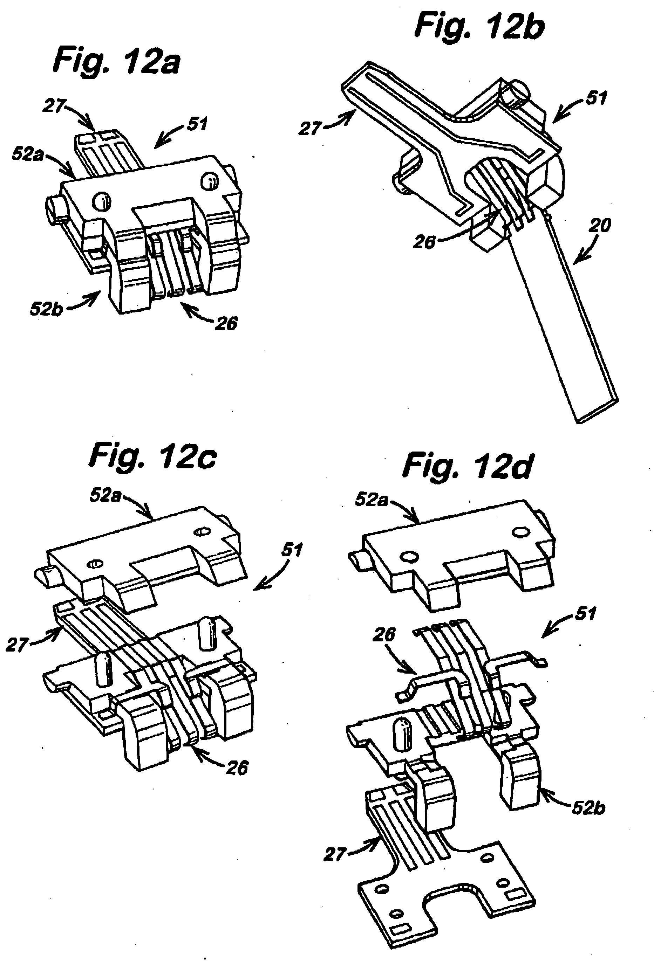

The adapter further includes a strip contact extension for facilitating in situ electrochemical analysis of the sample by connecting the test strip inserted into a test port to the attached glucose meter. The strip contact extension feature is illustrated in detail in Figures 12a, 12b, 12c and 12d. The strip connector 51 establishes electrical contact between the original test port of the glucose meter and the test strip 20 in the adapter. The electrical contact bars 26 on the strip connector contact electrodes formed on the test strip 20. The electrical contacts are connected to a printed circuit board 27 which contacts the original contacts (not shown) of the glucose meter. A substrate, comprising two parts 52a and 52b are used to assemble the electrical contact bars 26 and printed circuit board 27. The strip connector 51 is connected to the adapter such that the printed circuit board will contact the original contacts of the glucose meter when the adapter is attached to the glucose meter. The original contacts of the glucose meter connect to electronics located within the glucose meter housing. The test strip generates electrochemical signals that are passed by the strip connector to the glucose meter electronics. As known in the art, the electronics in the glucose meter process the signal and calculate the glucose level or other electrochemically detectable analyte of the blood or other interstitial fluid that is sampled by the testing device. The electronics transmit instructions for an appropriate display or output regarding the analysis.

The test strip 20 essentially comprises an electrochemical cell, including one or more working electrodes, which convert a chemical change produced by a reaction of glucose or other analyte in the blood sample to a current. The test strip 20 further includes a reference electrode as a standard to measure the potential of the working electrodes. Leads connect the electrodes to the contact bars 26 of the strip connector 51, which establishes electrical contact with the electronics of the glucose meter. The test strip thus generates a signal indicative of the level of glucose or other analyte in the blood and transmits this signal to the electronics of the device for processing. Those skilled in the art will recognize that a variety of test strip designs and configurations are available in accordance with the teachings of the present invention.

The illustrative embodiment of the invention achieves in situ testing of a blood sample based on proximity to a lancet wound. When the lancet 28 punctures the skin, a drop of blood forms on the skin surface. The test strip 20 is moved into close proximity to the puncture wound to ensure that blood contacts the strip. Moreover, the positioning of the test strip adjacent the lancet ensures that only small volumes of blood are required. According to the present invention, the test strip is separated from or positioned relative to the deployed lancet or lancing site between about 0.4 mm and about 1.3 mm, and preferably between about 0.7 mm and about 0.9 mm. Once the drop grows to a certain size, the drop touches the entrance of the capillary channel and is consequently drawn into the strip for analysis. The test strip wicks the blood sample directly from the puncture wound, allowing low volume blood samples for analysis. The test strip is positioned to automatically and efficiently direct the sample to a defined area of the test strip for analysis. The described arrangement efficiently conveys a sample from the skin to a precise position on the test strip with little to no losses. Furthermore, the established electrical connection between the test strip 20 and the glucose meter electronics, provided by the strip connector 51 of the adapter 10, allows analysis of the sample to occur without necessitating transfer of the sample or the test strip.

To measure blood glucose levels, a user first attaches the adapter 10 to the glucose meter 11 to provide an integrated sampling and testing device. The user inserts at test strip 20 into the test port, such that the test strip is in close proximity to the puncture site. The user cocks the lancing drive train by pulling back the cocking button 13, and subsequently releases the cocking button, which automatically returns to a rest position. The user selects a suitable penetration depth by rotating the depth adjustment button 18, the angular position of which defines the starting and stopping position of the lancet 28. The user then presses the adapter against a body part, such as a finger, such that the skin of the user contacts the pressure tip 16. The user depresses the trigger button 23 to release the lock on the lancet barrel 29 formed by the interface between the first rib 41 of the depth adjustment button 18 and the flexible arm 34 of the barrel. Consequently, the restoring force in the strong spring 30 drives the lancet tip 28 a predetermined depth into the skin, in close proximity to the test strip 20. The barrel base 33 abuts the interior rib 47 of the cocking button 13 to stop the lancet at the predetermined depth. The lancet assembly immediately retracts the lancet from the skin, yielding a drop of blood or other bodily fluid on the skin surface. According to one practice of the invention, the pressure tip 16 in the adapter cap 14 squeezes the skin to maximize the quantity of blood formed from a puncture. As the blood drop grows, the blood contacts a channel entry of the proximally located test strip 20, which absorbs the blood sample and directs the blood sample to an analysis portion. The analysis portion of the test strip generates a signal indicative of glucose levels, which is automatically provided to the glucose meter via the strip connector 51. In situ testing of the blood sample is enabled by virtue of the location of the adapter and test strip. In other words, the adapter allows the blood sample to be tested without necessitating manual transfer of the sample from the puncture site to the glucose meter. The glucose meter 11 displays and stores the glucose reading. After the analysis is complete, the user removes the adapter cap 14 and the lancet 28 from the lancet barrel 29. The user may then discard the lancet, if desired.

The illustrative embodiment of the invention provides significant advantages to testing and sampling blood by providing a device for integrating the sampling and testing of blood into a single, user-friendly device. The adapter can be retrofitted to an existing glucose meter to convert the glucose meter to have sampling capabilities. The adapter can also be detached and used separately from the glucose meter, if desired. The adapter encourages frequent use by reducing the pain, inconvenience and complexity of performing a blood test. The adapter reduces the number of accessories and steps by integrating all of the different accessories involved in glucose monitoring into one unit. The dedicated storage space provided in the adapter cap allows a user to store spare lancets, test strips and pressure tips conveniently and within easy reach. The invention further improves the efficiency and accuracy of testing by providing an automated transfer and analysis of the sample. The use of the clip-on adapter with a standard glucose meter eliminates the need to transfer a blood sample from a sampling site to a test strip and the need to then transfer the test strip to a separate glucose meter. The integrated testing device enabled by the adapter is compact, ergonomically sound, discrete and adjustable to different users and body parts while simultaneously providing fast and accurate results.

The present invention achieves pain-free testing in a number of ways. Shallower penetrations of the skin can be used to achieve a sufficient blood sample, reducing painful deep incisions in sensitive body parts. The present invention requires low sample volumes for analysis. The finger squeezing mechanism formed by the pressure tip on the lancet cap provides a high yield for small puncture wounds. The integrated sampling and testing feature further ensures full usage of the obtained sample and limits "leftovers" on the skin. In current systems, complex and inaccurate sample transfer from a sampling point to an application area on a test strip requires surplus sample due to poor utilization of an obtained sample drop. The present invention removes the inefficiency of transferring samples and provides optimal utilization of the obtained sample by automatically directing the sample to a precise location on the test strip. The superficial penetrations reduce agitation of nerve endings in the skin and reduce pain in sensitive body areas.

The variable depth of the lancet and the ability to test on a number of different body parts in addition to the finger reduces the concentration and repetition of micro-traumata in a small area, which result in tinting, itching, dried and callous skin areas. The illustrated depth adjustment mechanism, whether implemented in the adapter device or in a standard lancing device, provides a user-friendly means for precisely controlling the penetration depth of the lancet.

The present invention further provides easy and uncomplicated operation. The sampling and testing device significantly reduces the time and difficulty involved in sampling and testing blood. The adapter is designed such that one-handed operation is possible, eliminating the need for a separate workspace. The fully automated testing system is not subject to human error and inefficiency. The present invention also reduces waste by efficiently utilizing available resources. The present invention further protects against compromised test results due to contamination or an improperly calibrated glucose meter.

In conclusion, the integrated sampling and testing device of the present invention significantly reduces the obstacles associated with frequent glucose monitoring. The present invention promotes frequent monitoring for diabetic individuals by providing a simple, efficient, fast and accurate integrated sampling and testing device.

Since certain changes may be made in the above constructions without departing from the scope of the invention, it is intended that all matter contained in the above description or shown in the accompanying drawings be interpreted as illustrative and not in a limiting sense.

It is also to be understood that the following claims are to cover all generic and specific features of the invention described herein, and all statements of the scope of the invention which, as a matter of language, might be said to fall therebetween.

In addition, it will of course be understood that the present invention has been described above purely by way of example and modifications of detail can be made within the scope of the invention.

The following are numbered aspects of the invention which form part of the description and are not to be searched:-
1. An adapter for a testing device for testing analytes in bodily fluids comprising:
   a housing;
   a lancet assembly comprising a lancet drive train for holding and driving a lancet into the skin of a user;
   a test port for holding a test strip; and
   a housing connector for connecting the adapter housing to the testing device.
2. The adapter of claim 1, further comprising:
   a strip connector for establishing electrical contact between a test strip in the test port of the adapter and the testing device.
3. The adapter of claim 1 or claim 2, further comprising a removable lancet cap including a pressure ring to assist extraction of the blood sample.
4. The adapter of claim 3, further comprising a dedicated storage compartment.
5. The adapter of any one of claims 1 to 4, further comprising a cocking button for cocking the lancet drive train.
6. The adapter of any one of claims 1 to 5, further comprising a trigger button for actuating the lancet drive train.
7. The adapter of any one of claims 1 to 6, further comprising a depth adjustment button for adjusting a penetration depth of the lancet.
8. The adapter of claim 7, wherein the angular position of the depth adjustment button defines a cocked position and a final extended position of the lancet.
9. The adapter of claim 8, wherein the depth adjustment button includes a first slanted protrusion for defining the cocked position and a second slanted protrusion that is parallel to the first slanted protrusion for defining the final extended position.
10. The adapter of claim 9, wherein the first slanted protrusion contacts a stop on the lancet drive train to define a cocked position.
11. The adapter of claim 9 or claim 10, further comprising a cocking button for cocking the lancet drive train, wherein the second slanted protrusion contacts a fixed cocking rib on the cocking button.
12. The adapter of claim 11, wherein the cocking button includes a stop for the lancet drive train, such that the position of the cocking button defines the final extended position of the lancet.
13. The adapter of any one of the preceding claims, wherein the testing device is a glucose meter and the analyte being tested is glucose in a sample of blood.
14. A depth adjustment mechanism for a lancet device, comprising:
   a substrate movably mounted in a slot of a housing of the lancet device and having an interior surface;
   a first projection on the interior surface of the substrate and defining an interface with a lancet drive train; and
   a second projection on the interior surface of the substrate substantially parallel to the first projection and defining an interface with a cocking button.
15. The depth adjustment mechanism of claim 14, wherein the position of the substrate in the slot defines a cocked position of the lancet drive train and a stopping position of the lancet drive train.
16. The depth adjustment mechanism of claim 15, wherein the first projection interfaces with a catch on the lancet drive train to define the cocked position.
17. The depth adjustment mechanism of claim 15 or claim 16, wherein the second projection interfaces with a stop for the lancet drive train to define the stopping position.
18. The depth adjustment mechanism of claim 17, wherein the stop for the lancet drive train comprises a cocking button for the lancet drive train.
19. The depth adjustment mechanism of claim 18, further comprising a rim disposed on the interior side, wherein the rim interfaces with a groove on the housing to allow movement of the substrate.
20. The depth adjustment mechanism of any one of claims 14 to 19, further comprising a knob disposed on an external surface of the substrate to allow a user of the lancet device to move the substrate in the slot.
21. The depth adjustment mechanism of any one of claims 14 to 20, further comprising a cocking button for cocking the lancet drive train and including a cocking rib for interfacing with the second projection and an interior rib defining a stop for lancet drive train.
22. The depth adjustment mechanism of claim 21, further comprising a catch on the lancet drive train for interfacing with the first projection to define a cocked position of the lancet drive train.
23. An integrated testing device for testing analytes in bodily fluids comprising the depth adjustment mechanism according to any one of claims 14 to 22.
24. An integrated testing device for testing analytes in bodily fluids comprising:
   a housing;
   a lancet assembly comprising a lancet drive train for holding and driving a lancet into the skin of a user;
   a test port for holding a test strip;
   a testing device for testing analytes in bodily fluids applied to the test strip;
   and
   a housing connector for connecting the housing to the testing device.
25. A connector for connecting an adapter containing a lancing device to a testing device for testing analytes in bodily fluids comprising:
   a housing surface adapted to be removably mounted on the testing device; and
   a projection on the housing surface,
   wherein the projection is configured for connection with a housing feature on the testing device, such that the connector connects the adapter to the testing device.
26. A connector according to claim 25, wherein the housing surface is a battery door and the housing feature is an opening on the testing device.
27. A method of providing an integrated testing device, comprising:
   providing a testing device for testing analytes in bodily fluids;
   providing an adapter containing a housing, a lancet assembly, a test port for holding a test strip, a strip connector for establishing electrical contact between a test strip in the test port of the adapter and the testing device, and a housing connector for connecting the adapter housing to the testing device; and
   attaching said adapter to said testing device using the housing connector.
28. A method of sampling and testing a sample of bodily fluid comprising:
   providing a testing device for testing analytes in bodily fluids;
   providing an adapter containing a housing, a lancet assembly including a lancet, a test port for holding a test strip, a test strip disposed in the test port, a strip connector for establishing electrical contact between a test strip in the test port of the adapter and the testing device, and a housing connector for connecting the adapter housing to the testing device;
   attaching the adapter to the testing device using the housing connector to form an integrated device;
   cocking the lancet assembly; and
   actuating the lancet assembly to drive the lancet into the skin a predetermined depth to produce a sample of bodily fluid, wherein the test strip absorbs the sample and produces a signal indicative of an analyte level and wherein the strip connector automatically transmits the signal indicative of the analyte level to the testing device.

## Claims

1. A lancet device comprising:
a lancet (28) for puncturing the skin of a user;
a lancet drive train for holding and driving the lancet into the skin of a user, the lancer drive train having a cocked position and a stopping position;
a cocking button (13) for cocking the lancet drive train; and
a depth adjustment mechanism for adjusting the penetration depth of the lancet,
**characterized in that** the depth adjustment mechanism comprises:
a substrate (18) adapted to be movably mounted in a slot of a housing (12) of the lancet device and having an interior surface, wherein the position of the substrate in the slot defines the cocked position and the stopping position;
a first projection (41) on the interior surface of the substrate, the first projection adapted to interface with the lancet drive train to define the cocked position; and
a second projection (42) on the interior surface of the substrate substantially parallel to the first projection, the second projection adapted to interlace with a cocking button (13) to define the stopping position.

2. The lancet device of claim 1, wherein the first projection (41) is adapted to interface with a catch (35) on the lancet drive train.

3. The lancet device of claim 1 or claim 2, wherein the cocking button includes a stop and the second projection is adapted to interface with the stop (47).

4. The lancet device claim 1, wherein the depth adjustment mechanism further comprises a rim (44) disposed on the interior side, wherein the rim is adapted to interface with a groove on the housing to allow movement of the substrate.

5. The lancet device of any one of claims 1 to 4, wherein the depth adjustment mechanism further comprises a knob (40) disposed on an external surface of the substrate to allow a user of the lancet device to move the substrate in the slot.

6. The lancet device of any one of claims 1 to 5, wherein the cocking button includes a cocking rib (46) adapted to interface with the second projection and an interior rib (17) defining a stop for lancet drive train.

7. The lancet device claim 6, further comprising a catch (35) on the lancet drive train adapted to interface with the first projection to define a cocked position of the lancet drive train.

8. An integrated testing device for testing analytes in bodily fluids comprising the lancet device according to any one of claims 1 to 7.

9. An integrated testing device for testing analytes in bodily fluids comprising:
a housing (12);
a test port for holding a test strip (20);
a testing device for testing analyses in bodily fluids applied to the test strip;
a housing connector (17) for connecting the housing to the testing device; and
the lancet device according to any one of claims 1 to 7.

10. The device of claim 9, wherein the housing connector comprises:
a removeable housing surface; and
a projection (17a, 17b) on the housing surface,
wherein the projection is configured for connection with a housing feature (50a, 50b) on the testing device, such that the connector connects the adapter to the testing device.

## Patentansprüche

1. Lanzettenvorrichtung, die aufweist:
eine Lanzette (28) zum Durchstechen der Haut eines Benutzers;
einen Lanzetten-Antriebszug zum Halten und Treiben der Lanzette in die Haut eines Benutzers, wobei der Lanzetten-Antriebszug eine Spannposition und eine Halteposition hat;
einen Spannknopf (13) zum Spannen des Lanzetten-Antriebszuges; und
einen Tiefenanpassmechanismus zum Anpassen der Eindringtiefe der Lanzette,
**dadurch gekennzeichnet, dass** der Tiefenanpassmechanismus aufweist:
ein Substrat (18), das dazu ausgelegt ist, bewegbar in einem Schlitz eines Gehäuses (12) der Lanzettenvorrichtung angeordnet zu sein, und eine Innenfläche hat, wobei die Position des Substrats in dem Schlitz die Spannposition und die Halteposition definiert;
einen ersten Vorsprung (41) auf der Innenfläche des Substrats, wobei der erste Vorsprung dazu ausgelegt ist, an den Lanzetten-Antriebszug anzukoppeln, um die Spannposition zu definieren; und
einen zweiten Vorsprung (42) auf der Innenfläche des Substrates im Wesentlichen parallel zu dem ersten Vorsprung, wobei der zweite Vorsprung dazu ausgelegt ist, an einen Spannknopf (13) zu koppeln, um die Halteposition zu definieren.

2. Lanzettenvorrichtung nach Anspruch 1, bei der der erste Vorsprung (41) dazu ausgelegt ist, mit einer Arretierung (35) auf dem Lanzetten-Antriebszug zu koppeln.

3. Lanzettenvorrichtung nach Anspruch 1 oder Anspruch 2, bei der der Spannknopf ein Anschlagelement aufweist und der zweite Vorsprung so ausgelegt ist, dass er an das Anschlagelement (47) koppelt.

4. Lanzettenvorrichtung nach Anspruch 1, bei der der Tiefenanpassmechanismus weiter einen Kranz (44) aufweist, der auf der Innenseite angeordnet ist, wobei der Kranz dazu ausgelegt ist, in eine Nut auf dem Gehäuse zu koppeln, um die Bewegung des Substrates zu ermöglichen.

5. Lanzettenvorrichtung nach einem der Ansprüche 1 bis 4, bei der der Tiefenanpassmechanismus weiter einen Knopf (40) aufweist, der auf einer Außenfläche des Substrates angeordnet ist, um es einem Benutzer der Lanzettenvorrichtung zu ermöglichen, das Substrat in dem Schlitz zu bewegen.

6. Lanzettenvorrichtung nach einem der Ansprüche 1 bis 5, bei der der Spannknopf eine Spannrippe (46), die dazu ausgelegt ist, an den zweiten Vorsprung zu koppeln, und eine innere Rippe (47), die ein Anschlagelement für den Lanzetten-Antriebszug definiert, umfasst.

7. Lanzettenvorrichtung nach Anspruch 6, weiter mit einer Arretierung (35) auf dem Lanzetten-Antriebszug, die dazu ausgelegt ist, an den ersten Vorsprung zu koppeln, um eine Spannposition des Lanzetten-Antriebszuges zu definieren.

8. Integrierte Testvorrichtung zum Testen von Analyten in Körperfluiden, die die Lanzettenvorrichtung nach einem der Ansprüche 1 bis 7 aufweist.

9. Integrierte Testvorrichtung zum Testen von Analyten in Körperfluiden, die aufweist:
ein Gehäuse (12);
eine Testöffnung zum Halten eines Teststreifens (20),
eine Testvorrichtung zum Testen von Analyten in Körperfluiden, die mit dem Teststreifen verwendet wird;
einen Gehäuseverbinder (17) zum Verbinden des Gehäuses mit der Testvorrichtung; und
die Lanzettenvorrichtung nach einem der Ansprüche 1 bis 7.

10. Vorrichtung nach Anspruch 9, bei der der Gehäuseverbinder aufweist:
eine entfernbare Gehäusefläche; und
einen Vorsprung (17a, 17b) auf der Gehäusefläche,
wobei der Vorsprung für die Verbindung mit einem Gehäusemerkmal (50a, 50b) auf der Testvorrichtung ausgestaltet ist, so dass der Verbinder den Adapter mit der Testvorrichtung verbindet.

## Revendications

1. Dispositif de lancette comprenant :
■ une lancette (28) pour perforer la peau d'un utilisateur ;
■ un train d'entraînement de lancette pour maintenir et entraîner la lancette dans la peau d'un utilisateur, le train d'entraînement de lancette ayant une position armée et une position d'arrêt ;
■ un bouton d'armement (13) pour armer le train d'entraînement de lancette ; et
■ un mécanisme d'ajustement de profondeur pour ajuster la profondeur de pénétration de la lancette,
**caractérisé en ce que** le mécanisme d'ajustement de profondeur comprend :
■ un substrat (18) adapté pour être monté de manière amovible dans une fente d'un boîtier (12) du dispositif de lancette et ayant une surface intérieure, dans lequel la position du substrat dans la fente définit la position armée et la position d'arrêt ;
■ une première saillie (41) sur la surface intérieure du substrat, la première saillie étant adaptée pour s'interfacer avec le train d'entraînement de lancette afin de définir la position armée ; et
■ une seconde saillie (42) sur la surface intérieure du substrat sensiblement parallèle à la première saillie, la seconde saillie étant adaptée pour s'interfacer avec un bouton d'armement (13) pour définir la position d'arrêt.

2. Dispositif de lancette selon la revendication 1, dans lequel la première saillie (41) est adaptée pour s'interfacer avec une prise (35) sur le train d'entraînement de lancette.

3. Dispositif de lancette selon la revendication 1 ou la revendication 2, dans lequel le bouton d'armement comprend une butée et la seconde saillie est adaptée pour s'interfacer avec la butée (47).

4. Dispositif de lancette selon la revendication 1, dans lequel le mécanisme d'ajustement de profondeur comprend en outre un bord (44) disposé sur le côté intérieur, dans lequel le bord est adapté pour s'interfacer avec une rainure sur le boîtier pour permettre le mouvement du substrat.

5. Dispositif de lancette selon l'une quelconque des revendications 1 à 4, dans lequel le mécanisme d'ajustement de profondeur comprend en outre un bouton (40) disposé sur une surface extérieure du substrat pour permettre à un utilisateur du dispositif de lancette de déplacer le substrat dans la fente.

6. Dispositif de lancette selon l'une quelconque des revendications 1 à 5, dans lequel le bouton d'armement comprend une rainure d'armement (46) adaptée pour s'interfacer avec la seconde saillie et une nervure intérieure (47) définissant une butée pour le train d'entraînement de lancette.

7. Dispositif de lancette selon la revendication 6, comprenant en outre une prise (35) sur le train d'entraînement de lancette, adaptée pour s'interfacer avec la première saillie afin de définir une position armée du train d'entraînement de lancette.

8. Dispositif de test intégré pour tester des substances à analyser dans les fluides corporels comprenant le dispositif de lancette selon l'une quelconque des revendications 1 à 7.

9. Dispositif de test intégré pour tester des substances à analyser dans des fluides corporels comprenant :
■ un boîtier (12) ;
■ un orifice de test pour contenir une bande de test (20) ;
■ un dispositif de test pour tester des substances à analyser dans des fluides corporels, appliquées sur la bande de test ;
■ un connecteur de boîtier (17) pour raccorder le boîtier au dispositif de test ; et
■ le dispositif de lancette selon l'une quelconque des revendications 1 à 7.

10. Dispositif selon la revendication 9, dans lequel le connecteur de boîtier comprend :
■ une surface de boîtier amovible ; et
■ une saillie (17a, 17b) sur la surface de boîtier, dans lequel une saillie est configurée pour se raccorder avec une caractéristique de boîtier (50a, 50b) sur le dispositif de test, de sorte que le connecteur raccorde un adaptateur au dispositif de test.
